(19) Europäisches Patentamt
European Patent Office
Office européen des brevets

(11) Publication number: 0 539 480 B1

(12) # EUROPEAN PATENT SPECIFICATION

(45) Date of publication of patent specification: **15.11.95**

(51) Int. Cl.$^{6}$: **A61K 7/16**

(21) Application number: **91913575.6**

(22) Date of filing: **11.07.91**

(86) International application number:
**PCT/US91/04850**

(87) International publication number:
**WO 92/00721 (23.01.92 92/03)**

(54) **ANTICALCULUS/ANTIPLAOUE COMPOSITIONS USING AZACYCLOALKANE DIPHOSPHONATES.**

(30) Priority: **13.07.90 US 552399**

(43) Date of publication of application:
**05.05.93 Bulletin 93/18**

(45) Publication of the grant of the patent:
**15.11.95 Bulletin 95/46**

(84) Designated Contracting States:
**AT BE CH DE DK ES FR GB GR IT LI LU NL SE**

(56) References cited:
**FR-A- 2 361 865**
**US-A- 3 988 443**
**US-A- 4 575 456**

(73) Proprietor: **THE PROCTER & GAMBLE COMPANY**
**One Procter & Gamble Plaza**
**Cincinnati,**
**Ohio 45202 (US)**

(72) Inventor: **NELSON, Dennis, George, Anthony**
**8485 Rupp Farm Drive**
**West Chester, OH 45069 (US)**
Inventor: **SMITHERMAN, Herbert, Charles**
**1002 Redway Avenue**
**Cincinnati, OH 45229 (US)**

(74) Representative: **Brooks, Maxim Courtney et al**
**Procter & Gamble Limited**
**Whitley Road**
**Longbenton**
**Newcastle-upon-Tyne NE12 9TS (GB)**

EP 0 539 480 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to oral care compositions such as dentifrices, mouthwashes, lozenges, chewing gums, and the like, which are designed to prevent the accumulation of calculus, or "tartar", as it is sometimes called, and bacterial "plaque" on teeth.

Dental calculus is a deposit which forms on the surfaces of the teeth at the gingival margin. Supragingival calculus appears principally in the areas near the orifices of the salivary ducts; e.g., on the lingual surfaces of the lower anterior teeth and on the buccal surfaces of the upper first and second molars, and on the distal surfaces of the posterior molars.

Mature calculus consists of an inorganic material which is largely calcium phosphate arranged in a hydroxyapatite crystal lattice structure similar to bone, enamel and dentine. An organic portion is also present and consists of desquamated epithelial cells, leukocytes, salivary sediment, food debris and various types of microorganisms.

As the mature calculus develops, it becomes visibly white or yellowish in color unless stained or discolored by some extraneous agent. In addition to being unsightly and undesirable from an aesthetic standpoint, the mature calculus deposits are constant sources of irritation of the gingiva.

A wide variety of chemical and biological agents have been suggested in the art to retard calculus formation or to remove calculus after it is formed. Mechanical removal of this material periodically by the dentist is, of course, routine dental office procedure.

The chemical approach to calculus inhibition generally involves crystal growth inhibition and/or chelation of calcium ion which prevents the calculus from forming and/or breaks down mature calculus by removing calcium.

Dental plaque comprises an accumulation of bacteria and bacterial by-products on teeth. Plaque adheres tenaciously at points of irregularity or discontinuity, e.g., on rough calculus surfaces, at the gum line, and the like. The chemical approach to plaque inhibition generally involves the use of antibacterials.

US-A-4,885,155, to Parran and Sakkab, granted December 5, 1989, relates to oral compositions containing pyrophosphate salts which provide an anticalculus benefit.

As will be seen hereinafter from the discussion of the literature, there has been a continuing search for anticalculus agents. Unfortunately, the chemical composition of calculus is sufficiently similar to that of the healthy tooth mineral (primarily, calcium hydroxyapatite) that many materials which can attack and remove calculus would also undesirably attack the underlying tooth structure. This is particularly true with regard to many chelating agents which sequester calcium ions. There has also been a continuing search for effective antiplaque agents. Since both calculus and plaque affect oral health, it would be especially useful to provide compositions which provide both anticalculus and antiplaque benefits.

The present invention provides a new solution to the problem of dental calculus and dental plaque by means of the compositions disclosed hereinafter.

US-A-3,941,772, March 2, 1976, and US-A-3 988 443 relate to azacycloalkane diphosphonates and disclose their use to avoid the formation of tartar or plaque the latter optionally also in combination with an antiseptic, chlorothymol, in a mouthwash formulation. US-A-3,678,154, July 18, 1972 to Widder, et al. discloses oral compositions containing certain phosphonates and fluoride. US-A-3,737,522, June 5, 1973 to Francis discloses oral compositions containing certain carbonyl diphosphonates.

EP-A-0,251,591, published January 7, 1988 and GB-A-2,200,551, published August 10, 1988, relate to the use of TRICLOSAN plus pyrophosphate in oral care compositions.

Various references in addition to US-A-4,885,155, cited above, relate to anticalculus agents of various types. The prior art discloses a number of chelating agents for this purpose. GB-A-490,384, February 15, 1937, discloses oral compositions containing ethylenediaminetetraacetic acid, nitrilotriacetic acid and related compounds as anticalculus agents.

In addition to the above references, the prior art discloses dentifrices and mouthwashes containing soluble pyrophosphate salts which have been indicated for a variety of purposes. Included among such references are US-A-2,941,926, June 21, 1960 to Salzmann, et al. which discloses dental powders containing chlorophyll and pyrophosphate salts. US-A-3,137,632, June 16, 1964 to Schiraldi discloses toothpastes containing pyrophosphate salts. US-A-3,927,201 and US-A-3,927,202, December 16, 1975 to Baines, et al. and Harvey, et al., respectively, disclose toothpastes which utilize soluble pyrophosphates as abrasives. US-A-4,244,931, January 13, 1981 and US-A-4,247,5226, January 27, 1981 to Jarvis, et al. disclose pyrophosphate salts in dicalcium phosphate systems. Japanese Patent Application Disclosure No. 4945-1974 discloses soluble pyrophosphates in a variety of dentifrice systems. US-A-4,333,551, April 6, 1982 to Parran discloses tetraalkali metal salts in mouthwash compositions. Draus, Lesniewski and Miklos, *Pyrophosphate and Hexametaphosphate Effects In Vitro Calculus Formation*, Arch. Oral Biol., Vol. 15,

pp. 893-896 (1970), disclose the *in vitro* effectiveness of soluble pyrophosphate salts against calculus. However, they indicate that pyrophosphate efficacy would be inhibited by phosphatases *in vivo*.

The references suggesting that pyrophosphates could reduce calculus, but either suggesting problems associated with their use or not recognizing problems, are Rapp, G. W. et al, "Pyrophosphate: a factor in Tooth Erosion", J. D. Res. March-April 1960, Vol. 39, No. 2 pp. 372-376; the Draus article cited above; Briner et al, "In Vitro and In Vivo Evaluation of Anticalculus Agents", *Calc. Tiss. 11*, pp. 10-22 (1973); US-A-3,934,002, Jan. 20, 1976 to Haefele; and GB-A-490,384, Feb. 15, 1937.

US-A-4,847,070, July 11, 1989 to Pyrz et al. relates to oral compositions which are effective against calculus containing a chelating agent which is an acrylic acid polymer or copolymer or EDTA, together with a strontium source, a fluoride ion source and a pyrophosphate ion source.

US-A-4,661,341, April 28, 1987 to Benedict et al. relates to oral compositions containing an anticalculus agent which is an acrylic acid polymer or copolymer.

US-A-4,022,880, May 10, 1977 to Vinson et al. relates to compositions for inhibiting dental plaque and calculus formation comprising zinc ions and a nontoxic, organoleptically acceptable antibacterial agent.

GB-A-2,200,551, Gaffar, Nabi and Jannone, filed January 27, 1988, published August 10, 1988, relates to antibacterial, antiplaque and anticalculus oral compositions containing a linear molecularly dehydrated polyphosphate salt and a noncationic antibacterial agent.

US-A-4,656,031, April 7, 1987 to Lane et al. relates to a dentifrice which includes a surfactant and an antiplaque agent comprising a substantially water-insoluble noncationic antimicrobial agent or a zinc salt or a mixture thereof.

EP-A-0,251,591, Jackson et al., filed June 19, 1987 relates to oral hygiene compositions comprising specified pyrophosphates and antibacterials.

As noted above, US-A-4,885,155, December 5, 1989 to Parran, Jr. et al. relates to oral compositions containing particular pyrophosphate salts which provide an anticalculus benefit.

According to the present invention there is provided an oral care composition comprising:

(a) from 0.1% to 5% by weight of a source of an azacycloalkane-2,2-diphosphonate anion as an anticalculus agent;

(b) from 0.1% to 2% by weight of 5-chloro-2-(2,4-dichlorophenoxy)phenol; and

(c) a toxicologically acceptable oral carrier.

Preferably, the oral care compositions herein additionally comprise a course of an effective amount of fluoride ions. Other oral care adjunct materials such as pyrophosphate, various metal cations can optionally be used in the compositions herein. An oral care composition in the form of dentifrices, mouthwashes, lozenges and chewing gums are provided by this invention.

Preferred oral carriers assist in the emulsification or solubilization of the 5-chloro-2-(2,4-dichlorophenoxy)phenol. This can be achieved, for example, by inclusion of surfactants in the oral carrier.

Preferred compositions herein additionally comprise a source which will provide from 100 ppm to 1500 ppm of fluoride ions.

Highly preferred oral care compositions according to this invention additionally comprise a source of an effective amount of fluoride ions. Such sources can include, for example, sodium fluoride, stannous fluoride, sodium monofluorophosphate, and the like. Said source of fluoride ions preferably comprises from about 0.01% to about 1% by weight of the compositions herein. Dentifrice and mouthwash compositions containing the tartrate monosuccinate, tartrate disuccinate or mixtures thereof with fluoride sources are exemplified hereinafter.

Moreover, the oral care compositions according to this invention can also additionally comprise an effective amount of a source of pyrophosphate ions to provide additional calculus-controlling benefits.

In addition, oral care compositions herein can additionally comprise an effective amount of zinc citrate as an adjunct calculus-controlling agent.

Still other oral care compositions according to this invention can additionally comprise an effective amount of a source of a cation selected from zinc, indium, strontium and stannous cations, and mixtures thereof. Such compositions, or compositions which additionally comprise an effective amount of a member selected from sodium nitrate and potassium nitrate, are useful when treating sensitive teeth, e.g., in older patients.

Of course, since the compositions herein are intended for oral use, toxicologically-acceptable materials are used in the various compositions disclosed hereinafter.

All percentages, weights, ratios and proportions herein are by weight, unless otherwise specified.

The azacycloalkane diphosphonates used in the practice of this invention are known in the literature and their synthesis forms no part of this invention. Reference can be made to US-A-3,941,772, to Ploger et al, March 2, 1976, for syntheses of these materials.

In general, azacycloalkane-2,2-diphosphonates have the formula

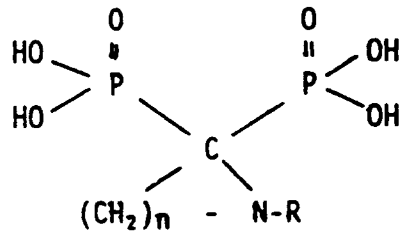

wherein R can be hydrogen or lower alkyl, e.g., methyl, ethyl, propyl, and n is an integer from 3 to 5. Such materials are prepared by reaction of the corresponding cyclic lactam with, for example, $H_3PO_3$. In this manner are prepared, for example, azacyclopentane-2,2-diphosphonic acid ("ACP"), N-methylazacyclopentane-2,2-diphosphonic acid ("NMAP") and azacycloheptane-2,2-diphosphonic acid, which is more properly named as 1-azacycloheptylidene-2,2-diphosphonic acid. Use of such materials as their acids or water-soluble salts, e.g., $Na^+$, $K^+$, $NH_4^+$ , salts, is contemplated by this invention. The sodium salts of 1-azacycloheptylidene-2,2-diphosphonic acid are referred to herein, collectively, as "AHP". (It will be appreciated that, as long as the salt is water soluble, the particular salt form used herein, i.e., mono-, di-, tri- or tetra-salt, is of no particular consequence in the practice of this invention, since it is the anion that provides the anticalculus benefit.) By "effective amount" of such diphosphonates herein is meant an amount sufficient to provide an anticalculus benefit.

The antimicrobial herein is TRICLOSAN (especially at levels of 0.3-0.6%) which is the trade name of 5-chloro-2-(2,4-dichlorophenoxy)phenol, also known as 2,4,4'-trichloro-2'-hydroxydiphenyl ether; see US-A-3,506,720. By "effective amount" of such antimicrobials herein is meant an amount sufficient to provide an antiplaque benefit.

The combination or conjoint use of AHP and TRICLOSAN constitutes a preferred embodiment of the present invention.

It is to be understood that the compositions and processes herein are designed to deliver an effective amount of the functional ingredients to the oral cavity safely and conveniently so that the desired anticalculus benefits are achieved. For most purposes, contacting the teeth which are prone to calculus and plaque accumulation with the AHP and TRICLOSAN for a period of from about 10 seconds to about 3 minutes once, or preferably three times, daily in an otherwise conventional oral care regimen of tooth brushing and/or use of mouthwash, will provide the desired benefits. Lesser or greater degrees of anticalculus benefits can be achieved by modifying the regimen. For example, use of a lozenge or chewing gum provides prolonged contact of the teeth with the functional ingredients.

The formulation of oral care compositions in the manner of this invention employs otherwise conventional ingredients such as well-known dentifrice abrasives, flavorants, thickening agents, fluid carriers (especially water-ethanol), sweetening agents, especially noncariogenic sweeteners such as the aspartic acid-derived sweeteners, saccharin and/or cyclamate, standard dental grade sources of fluoride ions such as sodium fluoride, stannous fluoride, and sodium monofluorophosphate (which are noted herein by way of exemplification, and not by way of limitation) and like materials which have come into broad usage in the dental arts. Various formularies are available and can be referred to for details of such materials.

In particular, the oral care compositions of this invention can comprise an effective amount of fluoride ions. By "effective amount" is meant sufficient fluoride ions to provide a dental enamel-strengthening or anticaries effect. Typically, compositions comprising from about 0.0025% to about 2%, preferably from about 0.01% to about 1% by weight of said fluoride ion source will provide sufficient fluoride ions for these desired benefits.

Highly preferred compositions herein comprise effective amounts of AHP plus TRICLOSAN plus fluoride.

Various other oral care adjunct materials can also be used herein. Such materials are, for example, pyrophosphate, zinc citrate, metal cations, and the like.

By an "effective amount" of a source of pyrophosphate ions herein is meant an amount which will provide adjunct anticalculus benefits. Typically, compositions can comprise from about 0.1% to about 10% by weight of pyrophosphate ions, which can be sourced from pyrophosphate salt such as tetrasodium, tetrapotassium, and disodium dihydrogen pyrophosphates.

By an "effective amount" of zinc citrate herein is meant an amount sufficient to provide adjunct anticalculus benefits. Typically, an amount of zinc citrate of from about 0.1% to about 5% by weight of the

compositions herein is sufficient.

By an "effective amount" of a source of cation, especially cations selected from zinc, indium, strontium and stannous cations, and mixtures thereof, herein is meant a sufficient amount of said cations to provide the benefits which are normally associated with the use of these particular materials in oral compositions. For example, the stannous cation has been associated with an anticaries benefit, as has the indium cation. Zinc and strontium cations have been noted for use in, for example, dentifrice compositions which are used in situations where the teeth have been made "sensitive" to pain, particularly in older teeth which have undergone serious erosion of the dental enamel. Typical usage levels to provide the aforesaid effective amount of such cations generally ranges from about 0.01% to about 3% by weight of the compositions. Materials such as indium chloride, stannous fluoride, strontium chloride, zinc chloride, and the like can be used for such purposes.

By an "effective amount" of sodium nitrate and potassium nitrate (preferred) herein is meant sufficient amounts of such materials to provide desensitization of otherwise sensitive teeth (as noted above). Typically, such amounts can comprise from about 0.01% to about 5% of the compositions herein.

Formulation of dentifrices typically involves the use of abrasives that are safe for use on teeth. Representative examples of abrasives useful herein include calcium carbonate, hydrated alumina, urea-formaldehyde resins (see US-A-3,070,510) and, as noted, the silica abrasives (see US-A-3,538,230 and US-A-3,862,307). "Syloid" silica xerogels, available from W. R. Grace and Company and precipitated silicas, e.g., "Zeodent 119" from J. M. Humber Corporation are a preferred class of abrasives used herein. Abrasives are typically in the 0.1 to 30 $\mu$m size, and are typically used in toothpastes at the 6% to about 80% level and in toothpowders at levels up to 99%. Silica abrasives are preferred herein.

Another embodiment of the present invention is a mouthwash composition. Conventional mouthwash compositions components can comprise the carrier for the actives of the present invention. Mouthwashes generally comprise from about 20:1 to about 2:1 of a water/ethyl alcohol solution and preferably other ingredients such as flavoring agents, sweeteners, humectants and sudsing agents such as those described above. The humectants, such as glycerin and sorbitol, give a moist feel to the mouth. Generally, on a weight basis the mouthwashes of the invention comprise from 5% to 60%, preferably from 10% to 25%, of ethyl alcohol ; from 0% to about 20%, preferably from 5% to 20%, of a humectant, from 0% to 2%, preferably from 0.01% to 0.15%, of an emulsifying agent; from 0% to 0.5% of a sweetening agent; from 0.03% to 0.3% of a flavoring agent; and the above-specified levels of AHP, TRICLOSAN, and fluoride.

Likewise, the formulation of chewing gums and lozenges presents no particular difficulties so long as toxicologically-acceptable carriers are used, as would naturally be the case in any product used in the oral cavity where ingestion of various ingredients by the user might occur. Suitable lozenge and chewing gum components are disclosed in US-A-4,083,955, issued April 11, 1978 to Grabenstetter et al. Suitable topical dental gels generally comprise a base of a humectant such as glycerin thickened with a suitable agent. Such gels generally do not contain an abrasive.

Flavoring agents can also be added to the compositions of the present invention. Suitable flavoring agents include oil of wintergreen, oil of peppermint, oil of spearmint, oil of sassafras and oil of clove. Sweetening agents are also useful and include aspartame, acesulfame, saccharin, dextrose, levulose and sodium cyclamate. Flavoring and sweetening agents are generally used in the compositions herein at levels of from 0.005% to 2% by weight.

The compositions of this invention may also contain emulsifying agents. Suitable emulsifying agents are those which are reasonably stable and foam throughout a wide pH range, and include non-soap anionic, nonionic, cationic, zwitterionic and amphoteric organic synthetic detergents. Many of these suitable surfactants are disclosed by Gieske et al. in US-A-4,051,234, issued September 27, 1977. It is preferred that the dentifrice compositions herein contain at least about 0.2%, preferably about 0.5% to about 6%, by weight of a surfactant, especially sodium $C_{12}$-$C_{18}$ alkyl sulfate, to help emulsify or partly dissolve TRICLOSAN.

Water may also be present in the compositions of this invention. Water employed in the preparation of commercially suitable compositions should preferably be deionized and free of organic impurities. Water generally comprises from 10% to 50% by weight, preferably from about 20% to about 40% by weight, of the toothpaste compositions herein. These amounts of water include the free water which is added plus that which is introduced with other materials, such as with sorbitol.

In preparing toothpastes, it is common to add some thickening material to provide a desirable consistency. Preferred thickening agents are carboxyvinyl polymers, carrageenan, hydroxyethyl cellulose and water soluble salts of cellulose ethers such as sodium carboxymethyl cellulose and sodium carboxymethyl hydroxyethyl cellulose. Natural gums such as gum karaya, gum arabic and gum tragacanth, and polysaccharide gums such as xanthan gum can also be used. Colloidal magnesium aluminum silicate can

be used as part of the thickening agent to further improve texture. Thickening agents in an amount from 0.5% to 5.0% by weight of the total composition may be used.

It is also desirable to include a humectant in a toothpaste to keep it from hardening. Suitable humectants include glycerin, sorbitol and other edible polyhydric alcohols at a level of from 10% to 70% by weight.

The pH of the present compositions and/or their pH in the mouth can be any pH which is safe for the mouth's hard and soft tissues. Such pH's are generally from 3 to 10, preferably from about 7 to about 9.

EXAMPLE I

A toothpaste composition is as follows.

| Ingredient | Percent (wt.) |
|---|---|
| Sorbitol | 24.0 |
| AHP (disodium salt) | 1.0 |
| TRICLOSAN | 0.3 |
| NaF | 0.24 |
| NaSaccharin | 0.45 |
| Polyethylene glycol | 3.0 |
| 10 Mol/L NaOH | to pH 7 |
| $TiO_2$ | 0.5 |
| FD&C Blue #1 | 0.05 |
| Silica | 21.7 |
| Glycerin | 8.9 |
| Xanthan gum | 0.6 |
| Carbopol | 0.2 |
| Flavor | 1.0 |
| Sodium alkyl sulfate (27.9% aqueous solution) | 6.5 |
| Water (deionized) | Balance |

EXAMPLE II

A mouthwash composition is as follows.

| Ingredient | Percent (wt.) |
|---|---|
| EtOH (190) by wt. | 8.5 |
| Sorbitol (70% aqueous solution) | 18.0 |
| AHP (trisodium salt) | 1.5 |
| TRICLOSAN | 0.6 |
| Polysorbate 80 | 0.6 |
| Dye (2% aqueous solution) | 0.07 |
| Pluronic F127 | 0.2 |
| Flavor | 0.075 |
| NaSaccharin | 0.04 |
| Sodium fluoride | 0.05 |
| 50% NaOH | to pH 7 |
| Water | Balance |

EXAMPLE III

A toothpowder composition is prepared by dry-blending ingredients, as follows:

| Ingredient | Percent (wt.) |
|---|---|
| AHP (monosodium salt) | 2.5 |
| Cetyl pyridinium chloride | 0.25 |
| Flavorant | 1.0 |
| Sodium alkyl sulfate | 6.0 |
| Silica xerogel (9 micron) | Balance |

EXAMPLE IV

A toothpaste composition is prepared according to Example I, but with the addition of 0.5% by weight of TDEPC in place of an equivalent weight of TRICLOSAN. The resulting formulation provides anticalculus, antiplaque and anticaries benefits.

EXAMPLE V

A lozenge comprising 80% maltose, 2% azacyclopentane-2,2-diphosphonic acid (triethanolammonium salt form), 1% sodium monofluorophosphate, 1.5% gum arabic, 0.1% strontium chloride, 0.1% flavorant, 0.05% magnesium stearate (tableting aid), the balance comprising corn starch, is prepared in a standard tablet press. In use, the lozenge is allowed to dissolve slowly in the mouth to bathe the teeth in the combination of active ingredients.

EXAMPLE VI

A chewing gum comprises 99.0% standard chewing gum base (chicle), 0.5% flavorant and 0.1% AHP, 0.1% NaF and 0.3% CPC.

EXAMPLE VII

A dentifrice powder comprises the following mixture of ingredients.

| Ingredient | Percent (wt.) |
|---|---|
| AHP | 1.5 |
| TRICLOSAN | 1.5 |
| Silica xerogel (as "Syloid") | 90.0 |
| Tetrasodium pyrophosphate | 4.0 |
| Sodium fluoride | 1.0 |
| Sodium alkyl sulfate ($C_{14}$) | 2.0 |

The composition of Example VII can be modified by replacing the tetrasodium pyrophosphate with an equivalent amount of tetrapotassium pyrophosphate.

EXAMPLE VIII

A mild toothpowder for sensitive teeth comprises 90% silica abrasive, 0.2% stannous fluoride, 0.1% potassium nitrate, 0.15% zinc sulfate, 1% sodium alkyl sulfate, 1% AHP (K salt), 1% TRICLOSAN, 0.25% CPC, the balance, corn starch. (per Reaction B, herein), the balance comprising flavorant.

EXAMPLE IX

An oral gel dentifrice comprises 3% AHP with 1.5% TRICLOSAN and 1.5% sodium lauryl sulfate, 1.5% Veegum, 1.5% carboxymethyl cellulose, 1.0% NaF, the balance comprising water.

EXAMPLE X

Base toothpaste compositions comprising standard amounts and types of abrasives, thickeners, humectants, surfactants and flavorants and prepared and blended with the following combinations of ingredients to provide dentifrice compositions A through E as follows.

| Ingredient (wt. ratio) | Percent (wt.) in Dentifrice |
|---|---|
| A. Zinc citrate/AHP/TRICLOSAN (1:1:1) | 10 |
| B. KF/PAM/TDEPC/ACP (1:0.1:0.1:5) | 15 |
| C. Sodium peroxide/AHP | 5 |
| D. Indium trichloride/CPC/NMAP (0.2:1:1) | 5 |
| E. TRICLOSAN/tetrasodium pyrophosphate/AHP (0.5:1:1) | 10 |

While the foregoing illustrates not only the use of the basic compositions of this invention and several embodiments thereof, but also various combinations of the herein-disclosed active ingredients with various adjunct agents for oral care, it is to be understood that such adjunct agents are given by way of exemplification and not by way of limitation. Other adjunct oral care agents can be used in such compositions, including materials such as: the ethylenediamine tetraacetates (EDTA) generally at effective levels of 0.1%, or less; peroxides, especially 1% aqueous hydrogen peroxide; sodium tripolyphosphate (STPP), typically at 0.5%-10% levels, and the like. The following examples provide further guidance to the formulator.

EXAMPLE XI

Base mouthwash compositions comprising water, 15% ethanol, standard flavorants and dyes are prepared and blended with the following combinations of ingredients to provide mouthwash compositions A, B and C, as follows.

| Ingredient (wt. ratio) | Percent (wt.) in Mouthwash |
|---|---|
| A. $H_2O_2$/AHP (1:1) | 2.0 |
| B. EDTA/AHP/CPC (0.1:1:0.5) | 2.0 |
| C. STPP/AHP/CPC (10:1:0.5) | 5.0 |

As can be seen from the foregoing, a wide variety of compositions useful for treating teeth in patients who are susceptible to dental calculus formation and in need of such treatment are provided by the practice of this invention. It will also be appreciated that "multiple" compositions can be used in conjunction with each other, e.g., a toothpaste comprising AHP plus a separate toothpaste, mouthwash or spray comprising TRICLOSAN, can be separately applied to the teeth to afford multiple benefits. Such separate usage would not depart from the spirit and scope of this invention.

## Claims

**1.** An oral care composition, comprising:

(a) from 0.1% to 5% by weight of a source of an azacycloalkane-2,2-diphosphonate anion as an anticalculus agent;

(b) from 0.1% to 2% by weight of 5-chloro-2-(2,4-dichlorophenoxy)phenol; and

(c) a toxicologically acceptable oral carrier.

2. A composition according to Claim 1 wherein said anticalculus agent (a) is a salt of 1-azacycloheptylidene-2,2-diphosphonate.

3. An oral care composition according to Claim 1 which additionally comprises a source to provide from 100 ppm to 1500 ppm of fluoride ions.

4. An oral care composition according to Claim 1 in the form of a dentifrice or a mouthwash.

5. A composition according to Claim 1 wherein said oral carrier assists in the emulsification or solubilization of the 5-chloro-2-(2,4-dichlorophenoxy)phenol.

6. An oral care composition according to Claim 1 which additionally comprises a member selected from a source of pyrophosphate ions, a metal cation selected from zinc, indium, strontium and stannous cations, sodium nitrate, potassium nitrate, or mixtures thereof.

## Patentansprüche

1. Mundpflegezusammensetzung, welche enthält:
   (a) 0,1 Gew.-% bis 5 Gew.-% einer Quelle für ein Azacycloalkan-2,2-diphosphonatanion als Antizahnsteinmittel;
   (b) 0,1 Gew.-% bis 2 Gew.-% 5-Chlor-2-(2,4-dichlorphenoxy)phenol; und
   (c) einen toxikologisch annehmbaren, oralen Träger.

2. Zusammensetzung nach Anspruch 1, wobei das Antizahnsteinmittel (a) ein Salz von 1-Azacycloheptyliden-2,2-diphosphonat ist.

3. Mundpflegezusammensetzung nach Anspruch 1, welche zusätzlich eine Quelle enthält, welche 100 ppm bis 1500 ppm Fluoridionen liefert.

4. Mundpflegezusammensetzung nach Anspruch 1, in der Form einer Zahnpaste oder eines Mundwassers.

5. Zusammensetzung nach Anspruch 1, worin der orale Träger das Emulgieren oder Solubilisieren des 5-Chlor-2-(2,4-dichlorphenoxy)phenols unterstützt.

6. Mundpflegezusammensetzung nach Anspruch 1, welche zusätzlich einen Bestandteil enthält, der aus einer Quelle für Pyrophosphationen, einem Metallkation, das seinerseits aus Zink-, Indium-, Strontium- und Zinn(II)-Kationen ausgewählt ist, Natriumnitrat, Kaliumnitrat oder Gemischen hievon ausgewählt ist.

## Revendications

1. Composition pour les soins buccaux, comprenant :
   (a) de 0,1 à 5 % en poids d'une source d'anion azacycloalcane-2,2-diphosphonate servant d'agent antitartre ;
   (b) de 0,1 à 2 % en poids de 5-chloro-2-(2,4-dichlorophénoxy)phénol ; et
   (c) un véhicule pour utilisation orale acceptable d'un point de vue toxicologique.

2. Composition selon la revendication 1, dans laquelle ledit agent anti-tartre (a) est un sel 1-azacycloheptylidène-2,2-diphosphonate.

3. Composition pour les soins buccaux selon la revendication 1, qui comprend de plus une source suffisante pour délivrer de 100 à 1500 ppm d'ions fluor.

4. Composition pour les soins buccaux selon la revendication 1, se présentant sous la forme d'un dentifrice ou d'un bain de bouche.

5. Composition selon la revendication 1, dans laquelle ledit véhicule oral facilite l'émulsification ou la solubilisation du 5-chloro-2-(2,4-dichlorophénoxy)phénol.

6. Composition pour les soins buccaux selon la revendication 1, qui comprend de plus un élément choisi parmi une source d'ions pyrophosphate, un cation métallique choisi parmi les cations zinc, indium, strontium et étain(II), le nitrate de sodium, le nitrate de potassium, ou leurs mélanges.